# EUROPEAN PATENT APPLICATION

(11) **EP 3 450 541 A1**
(43) Date of publication of application: **06.03.2019**
(21) Application number: 17789660.2
(22) Date of filing: 27.04.2017
(51) Int. Cl.: C12N 5/00, C12N 5/0735, C12N 5/10

(54) **PURIFICATION METHOD FOR PANCREATIC PRECURSOR CELLS DERIVED FROM PLURIPOTENT STEM CELLS AND AMPLIFICATION METHOD THEREFOR**

(30) Priority: 28.04.2016 JP 2016091116
(71) Applicant: Takeda Pharmaceutical Company Limited, Osaka-shi, Osaka 541-0045 (JP)
(72) Inventor: IWATA, Hiroo, Kyoto-shi Kyoto 606-8501 (JP); KONAGAYA, Shuhei, Kyoto-shi Kyoto 606-8501 (JP)
(74) Representative: Jones, Nicholas Andrew
(86) International application number: PCT/JP2017/016728
(87) International publication number: WO 2017/188378

(57) **Abstract**

Disclosed are a method for culturing pancreatic progenitor cells derived from pluripotent stem cells, the method comprising step (A) of three-dimensionally culturing pancreatic progenitor cells derived from pluripotent stem cells in a medium containing a factor belonging to the epidermal growth factor (EGF) family and/or a factor belonging to the fibroblast growth factor (FGF) family, and (2) a Wnt agonist; a method for producing islet cells from pancreatic progenitor cells derived from pluripotent stem cells, the method comprising step (E) of inducing the differentiation of pancreatic progenitor cells cultured by the above method into islet cells; and a method for cryopreserving pancreatic progenitor cells derived from pluripotent stem cells, the method comprising step (F) of freezing pancreatic progenitor cells cultured by the above method.

## Description

### Technical Field

The present invention relates to a method for culturing pancreatic progenitor cells derived from pluripotent stem cells, a method for producing islet cells from pancreatic progenitor cells derived from pluripotent stem cells, and a method for cryopreserving pancreatic progenitor cells derived from pluripotent stem cells. Further, the present invention relates to a medium for culturing pancreatic progenitor cells derived from pluripotent stem cells.

### Background of Invention

Pancreas transplantation and pancreatic islet transplantation are effective as therapeutic methods for diabetes (particularly insulin-dependent diabetes); however, the small number of organ donations, the need to take immunosuppressants for inhibiting immunological rejection, etc., become major issues. Therefore, in order to solve such problems, studies for inducing differentiation into islet cells from pluripotent stem cells, such as induced pluripotent stem cells (iPS cells) and embryonal stem (ES) cells, and pancreatic progenitor cells isolated from organisms have been widely performed using cells derived from mice and humans (for example, NPL 1).

To obtain islet cells, pluripotent stem cells are allowed to differentiate into pancreatic progenitor cells through mesendoderm cells and definitive endoderm, and then differentiate into endocrine precursor cells and islet cells, such as α-cells, β-cells, and δ-cells. Although many islet cells are required for pancreatic islet transplantation, differentiated islet cells have low proliferation potential. In contrast, undifferentiated pluripotent stem cells have proliferation potential; however, induction of their differentiation requires a long period of time. In addition, when pluripotent stem cells are mixed into islet cells used for transplantation, there is a concern about tumor formation after transplantation. Accordingly, there are many research reports attempting proliferation of pancreatic progenitor cells derived from pluripotent stem cells.

In these studies, in order to proliferate pancreatic progenitor cells derived from pluripotent stem cells, co-culture is performed using various feeder cells (for example, NPL 2 and NPL 3). However, use of such feeder cells, and use of serum mean use of materials with unknown components, and there is a problem in that it is difficult to stably prepare pancreatic progenitor cells having uniform characteristics due to the difference in components among lots. Moreover, when feeder cells and serum derived from heterologous animals are used, there is a risk that they can be a source of infection with unknown pathogens derived from the heterologous animals.

NPL 2 reports that progenitor cells derived from ES cells were proliferated, without differentiation, by co-culturing the progenitor cells with mesenchymal cells.

NPL 3 reports that pancreatic progenitor cells derived from ES cells were proliferated, without differentiation, by co-culture with endothelial cells or culture in the presence of EGFL7; however, their proliferation potential is insufficient.

It is also reported that pancreatic progenitor cells isolated from an organism are proliferated by ex vivo culture; however, sufficient proliferation is not achieved in the case of pancreatic progenitor cells that are not derived from pluripotent stem cells.

For example, PTL 1 reports culture of pancreatic tissue fragments isolated from an organism using a specific cell culture medium. However, PTL 1 does not disclose use of pancreatic progenitor cells derived from pluripotent stem cells, and the cell population used for culture is not a homogeneous cell population. Various cell populations derived from pancreatic tissue are cultured, and PTL 1 does not disclose culture of a homogeneous pancreatic progenitor cell population.

### Citation List

### Patent Literature

PTL 1: JP5722835B

### Non-patent Literature

NPL 1: A Rezania et al. Nat Biotechnol. 2014 Nov; 32 (11): 1121-1133
NPL 2: JB Sneddon et al. Nature. 2012 Nov; 491 (7426): 765-768 NPL 3: DI Kao et al. Stem Cell Reports. 2015 Feb; 4 (2): 181-189

### Summary of Invention

### Technical Problem

An object of the present invention is to provide a method for culturing pancreatic progenitor cells derived from pluripotent stem cells, whereby pancreatic progenitor cells derived from pluripotent stem cells can be efficiently proliferated while suppressing their differentiation. Another object of the present invention is to provide a method for producing islet cells from pancreatic progenitor cells derived from pluripotent stem cells obtained by this culture method, and a method for cryopreserving pancreatic progenitor cells derived from pluripotent stem cells. Still another object of the present invention is to provide a culture medium that can efficiently proliferate pancreatic progenitor cells derived from pluripotent stem cells, while suppressing their differentiation.

### Solution to Problem

As a result of extensive studies to achieve the above objects, the present inventors found that the above objects can be achieved by culturing pancreatic progenitor cells derived from pluripotent stem cells in the form of cell aggregates in a medium containing an epidermal growth factor (EGF) and R-spondin 1, or a medium containing various combinations of FGF-7 and a GSK inhibitor (CHIR99021).

The present invention has been completed upon further examination based on these findings. The present invention provides a method for culturing pancreatic progenitor cells derived from pluripotent stem cells, a method for producing islet cells from pancreatic progenitor cells derived from pluripotent stem cells, a method for cryopreserving pancreatic progenitor cells derived from pluripotent stem cells, a medium for culturing pancreatic progenitor cells derived from pluripotent stem cells, etc., described below. In the following, the description "(I-1) to" includes (I-1-A), (I-1-B), etc., and the same applies to the others.

### (I) Method for culturing pancreatic progenitor cells derived from pluripotent stem cells

(I-1) A method for culturing pancreatic progenitor cells derived from pluripotent stem cells, the method comprising step (A) of three-dimensionally culturing pancreatic progenitor cells derived from pluripotent stem cells in a medium containing (1) a factor belonging to the epidermal growth factor (EGF) family and/or a factor belonging to the fibroblast growth factor (FGF) family, and (2) Wnt agonist.
   (I-1-A) The method according to (I-1), wherein the Wnt agonist is a factor belonging to the Wnt family, a factor belonging to the R-spondin family, norrin, and/or a GSK inhibitor.
   (I-1-B) The method according to (I-1), wherein the Wnt agonist comprises at least one factor selected from the group consisting of Wnt-1/Int-1, Wnt-2/Irp, Wnt-2b/13, Wnt-3/Int-4, Wnt-3a, Wnt-4, Wnt-5a, Wnt-5b, Wnt-6, Wnt-7a, Wnt-7b, Wnt-8a/8d, Wnt-8b, Wnt-9a/14, Wnt-9b/14b/15, Wnt-10a, Wnt-10b/12, Wnt-11, Wnt-16, CHIR99021, SB216763, SB415286, A1070722, BIO, BIO-acetoxime, Indirubin-3'-oxime, NSC 693868, TC-G 24, TCS 2002, TWS 119, siRNA, lithium, kenpaullone, R-spondin 1, R-spondin 2, R-spondin 3, R-spondin 4, and norrin.
(1-2) The method according to (I-1), wherein the Wnt agonist is a protein belonging to the R-spondin family and/or a GSK inhibitor.
   (I-2-A) The method according to (I-1), (I-1-A), (I-1-B), or (1-2), wherein the factor belonging to the EGF family and/or the factor belonging to the FGF family (1) is a factor binding to ErbB1 and/or a factor binding to FGFR2IIIb.
   (I-2-B) The method according to (I-1), (I-1-A), (I-1-B), or (1-2), wherein the factor belonging to the EGF family and/or the factor belonging to the FGF family (1) comprises at least one factor selected from the group consisting of EGF, a transforming growth factor α (TGF-α), amphiregulin, a heparin-binding EGF-like growth factor, a schwannoma-derived growth factor, betacellulin, a poxvirus growth factor, acidic fibroblast growth factors (aFGF, FGF-1), basic fibroblast growth factors (bFGF, FGF-2), FGF-3, keratinocyte growth factors (KGF, FGF-7), FGF-10, and FGF-22.
(1-3) The method according to (I-1), wherein the factor belonging to the EGF family and/or the factor belonging to the FGF family (1) is EGF, and the Wnt agonist (2) is R-spondin 1.
(1-4) The method according to (1-2), wherein the factor belonging to the EGF family is EGF, the factor belonging to the FGF family is FGF-7, the protein belonging to the R-spondin family is R-spondin 1, and the GSK inhibitor is CHIR99021.
(1-5) The method according to any one of (I-1) to (1-4), wherein the medium is a serum-free medium.
(1-6) The method according to any one of (I-1) to (1-5), wherein the culture is culture in the absence of feeder cells.
(1-7) The method according to any one of (I-1) to (1-6), wherein the pluripotent stem cells are iPS cells or ES cells.
(1-8) The method according to any one of (I-1) to (1-7), wherein the pluripotent stem cells are derived from a human.
(1-9) The method according to any one of (I-1) to (1-8), wherein the three-dimensional culture is suspension culture of aggregates of pancreatic progenitor cells.
(I-10) The method according to any one of (I-1) to (I-9), further comprising step (B) of further subculturing the pancreatic progenitor cells obtained in step A.
(I-11) The method according to any one of (I-1) to (I-10) for use in purification of pancreatic progenitor cells.
(I-12) The method according to any one of (I-1) to (I-11), further comprising step (C) of preparing iPS cells, wherein pancreatic progenitor cells derived from the iPS cells are used in step A.
(I-13) The method according to any one of (I-1) to (I-12), further comprising step (D) of inducing the differentiation of pluripotent stem cells into pancreatic progenitor cells, wherein the pancreatic progenitor cells are used in step A.

### (II) Method for producing islet cells from pancreatic progenitor cells derived from pluripotent stem cells

(II-1) A method for producing islet cells from pancreatic progenitor cells derived from pluripotent stem cells, the method comprising step (E) of inducing the differentiation of pancreatic progenitor cells cultured by the method according to any one of (I-1) to (1-13) into islet cells.

### (III) Method for cryopreserving pancreatic progenitor cells derived from pluripotent stem cells

(III-1) A method for cryopreserving pancreatic progenitor cells derived from pluripotent stem cells, the method comprising step (F) of freezing pancreatic progenitor cells cultured by the method according to any one of (I-1) to (1-13).

### (IV) Medium for culturing pancreatic progenitor cells derived from pluripotent stem cells

(IV-1) A medium for culturing pancreatic progenitor cells derived from pluripotent stem cells, the medium containing (1) a factor belonging to the EGF family and/or a factor belonging to the FGF family, and (2) Wnt agonist.
   (IV-1-A) The medium according to (IV-1), wherein the Wnt agonist is a factor belonging to the Wnt family, a factor belonging to the R-spondin family, norrin, and/or a GSK inhibitor.
   (IV-1-B) The medium according to (IV-1), wherein the Wnt agonist comprises at least one factor selected from the group consisting of Wnt-1/Int-1, Wnt-2/Irp, Wnt-2b/13, Wnt-3/Int-4, Wnt-3a, Wnt-4, Wnt-5a, Wnt-5b, Wnt-6, Wnt-7a, Wnt-7b, Wnt-8a/8d, Wnt-8b, Wnt-9a/14, Wnt-9b/14b/15, Wnt-10a, Wnt-10b/12, Wnt-11, Wnt-16, CHIR99021, SB216763, SB415286, A1070722, BIO, BIO-acetoxime, Indirubin-3'-oxime, NSC 693868, TC-G 24, TCS 2002, TWS 119, siRNA, lithium, kenpaullone, R-spondin 1, R-spondin 2, R-spondin 3, R-spondin 4, and norrin.
(IV-2) The medium according to (IV-1), wherein the Wnt agonist is a protein belonging to the R-spondin family and/or a GSK inhibitor.
   (IV-2-A) The medium according to (IV-1), (IV-1-A), (IV-1-B), or (IV-2), wherein the factor belonging to the EGF family and/or the factor belonging to the FGF family (1) is a factor binding to ErbB1 and/or a factor binding to FGFR2IIIb.
   (IV-2-B) The medium according to (IV-1), (IV-1-A), (IV-1-B), or (IV-2), wherein the factor belonging to the EGF family and/or the factor belonging to the FGF family (1) comprises at least one factor selected from the group consisting of EGF, a transforming growth factor α (TGF-α), amphiregulin, a heparin-binding EGF-like growth factor, a schwannoma-derived growth factor, betacellulin, a poxvirus growth factor, acidic fibroblast growth factors (aFGF, FGF-1), basic fibroblast growth factors (bFGF, FGF-2), FGF-3, keratinocyte growth factors (KGF, FGF-7), FGF-10, and FGF-22.
(IV-3) The medium according to (VI-1), wherein the factor belonging to the EGF family and/or the factor belonging to the FGF family (1) is EGF, and the Wnt agonist (2) is R-spondin 1.
(IV-4) The medium according to (IV-2), wherein the factor belonging to the EGF family is EGF, the factor belonging to the FGF family is FGF-7, the protein belonging to the R-spondin family is R-spondin 1, and the GSK inhibitor is CHIR99021.
(IV-5) The medium according to any one of (IV-1) to (IV-4), which is free of serum.
(IV-6) The medium according to any one of (IV-1) to (IV-5) for culture in the absence of feeder cells.
(IV-7) The medium according to any one of (IV-1) to (IV-6), wherein the pluripotent stem cells are iPS cells or ES cells.
(IV-8) The medium according to any one of (IV-1) to (IV-7), wherein the pluripotent stem cells are derived from a human.
(IV-9) The medium according to any one of (IV-1) to (IV-8), further containing at least one member selected from the group consisting of a Sonic Hedgehog signal inhibitor, a TGF-β receptor inhibitor, and retinoic acid.
(IV-10) The medium according to any one of (IV-1) to (IV-9) for use in the purification of pancreatic progenitor cells.

### (V) Use of pancreatic progenitor cells derived from pluripotent stem cells in a culture medium

(V-1) Use of a component according to any one of (IV-1) to (IV-4) and (IV-9) in a medium for culturing pancreatic progenitor cells derived from pluripotent stem cells.

### (VI) Pharmaceutical preparation

(VI-1) A pharmaceutical preparation comprising pancreatic progenitor cells cultured by the method according to any one of (I-1) to (I-13).

### (VII) Culture

(VII-1) An isolated culture comprising (1) a factor belonging to the EGF family and/or a factor belonging to the FGF family, (2) Wnt agonist, and 5 mass% or more, 10 mass% or more, 15 mass% or more, or 20 mass% or more of pancreatic progenitor cells.

### Advantageous Effects of Invention

According to the culture method of the present invention, it is possible to efficiently proliferate pancreatic progenitor cells derived from pluripotent stem cells, which are a highly homogeneous cell population, while suppressing their differentiation, under serum-free and feeder cell-free conditions. Since culture is performed under serum-free and feeder cell-free conditions, the difference in medium among lots can be reduced, and pancreatic progenitor cells with stable quality can be prepared.

Moreover, since the culture method of the present invention can be used for subcultures, it is possible to proliferate large amounts of pancreatic progenitor cells, and the pancreatic progenitor cells can be purified in the subculture process.

Furthermore, the pancreatic progenitor cells proliferated by the culture method of the present invention can be subjected to differentiation induction into islet cells, including insulin-producing cells (β-cells).

The pancreatic progenitor cells proliferated by the culture method of the present invention can be cryopreserved, and can also be proliferated even after thawing, as with before freezing.

Since large amounts of pancreatic progenitor cells can be proliferated by the culture method of the present invention, the culture method is advantageous in that it is possible to screen cells (for example, eliminate undifferentiated cells or tumorigenic cells) and to evaluate safety at the stage of pancreatic progenitor cells; in that the production time and production cost of pancreatic progenitor cells can be reduced; and in that large amounts of islet cells with guaranteed quality can be supplied for a short period of time.

The pancreatic progenitor cells proliferated by the culture method of the present invention, or islet cells obtained by inducing the differentiation of the pancreatic progenitor cells proliferated by the culture method of the present invention are useful as cell pharmaceutical preparations or devices for treating (type 1 or type 2) diabetes in such a manner that they are implanted in the affected area directly or after being encapsulated.

### Brief Description of Drawings

Fig. 1 shows the results of flow cytometry analysis after differentiation induction into pancreatic progenitor cells.
Fig. 2 shows photographs showing phase-contrast microscope images of cells cultured for 6 days in the presence of each factor or a combination thereof after differentiation induction into pancreatic progenitor cells.
Fig. 3 is a graph showing changes in the number of cells with time when cells are cultured in the presence of each factor or a combination thereof after differentiation induction into pancreatic progenitor cells. The vertical axis represents a relative value of the number of cells when the culture starting time is regarded as 1.
Fig. 4 shows the results of flow cytometry analysis of cells (not passaged) cultured for 6 days in the presence of each factor or a combination thereof after differentiation induction into pancreatic progenitor cells.
Fig. 5 is a graph showing changes in the number of cells with time when cells are adhesion-cultured after differentiation induction into pancreatic progenitor cells. The vertical axis represents a relative value of the number of cells when the culture starting time is regarded as 1.
Fig. 6 is a graph showing the rate (%) of SOX9- and BrdU-positive cells in each passage.
Fig. 7 shows the results of flow cytometry analysis of cells in each passage.
Fig. 8 is a graph showing the rate (%) of SOX9- and PDX1-positive cells in each passage.
Fig. 9 shows photographs showing phase-contrast microscope images of cell aggregates after 5 passages.
Fig. 10 is a graph showing changes in the number of cells with time when cells are cultured by passage at intervals of six days after differentiation induction into pancreatic progenitor cells. The vertical axis represents a relative value of the number of cells when the culture starting time is regarded as 1.
Fig. 11 shows photographs showing immunostaining images of cells after 9 passages.
Fig. 12 shows the results of flow cytometry analysis of cells after 9 passages.
Fig. 13 shows the results of flow cytometry analysis of cells after differentiation induction into islet cells.
Fig. 14 is a graph showing the results of a glucose tolerance test on cells after differentiation induction into islet cells. The vertical axis represents the amount of C-peptide secreted (pM/256 aggregates, 0.5 mL, 0.5 h).
Fig. 15 shows photographs showing immunostaining images of cells after differentiation induction into islet cells.
Fig. 16 is a graph showing the cell survival rate (%) when pancreatic progenitor cells are frozen using various cryopreservation solutions, stored at -196°C, and then thawed.
Fig. 17 is a graph showing changes in the number of cells with time when cells obtained by thawing cryopreserved cells, and non-cryopreserved cells are cultured in media for proliferating pancreatic progenitor cells. The vertical axis represents a relative value of the number of cells when the culture starting time is regarded as 1.
Fig. 18 is a graph showing changes in the number of cells with time when pancreatic progenitor cells derived from RPChiPS771-2 line are cultured with addition of four factors (EGF + RSPD1 + FGF-7 + CHIR99021). The vertical axis represents a relative value of the number of cells when the culture starting time is regarded as 1.
Fig. 19 shows the results of flow cytometry analysis when pancreatic progenitor cells derived from each human iPS cell line are cultured with the addition of four factors (EGF + RSPD1 + FGF-7 + CHIR99021).
Fig. 20 is a graph showing changes in the number of cells with time when pancreatic progenitor cells derived from each human iPS cell line are cultured with the addition of two to four factors. The vertical axis represents a relative value of the number of cells when the culture starting time is regarded as 1.
Fig. 21 is a graph showing the rate (%) of Ki67- and PDX1-positive cells when pancreatic progenitor cells derived from each human iPS cell line are cultured with the addition of two to four factors.

The present specification includes the contents described in the specification of Japanese Patent Application No. 2016-091116 (filed on April 28, 2016), on which the priority of the present application is based.

### Description of Embodiments

The present invention is described in detail below.

In the present specification, the terms "contain" and "comprise" include the meaning of "essentially consist of" and the meaning of "consist of."

In the present specification, the term "culture" refers to maintenance or/and proliferation of cells in an in-vitro environment. The term "culturing" refers to maintaining or/and proliferating cells outside tissue or outside the body (e.g., in a cell culture dish or flask).

### Pluripotent Stem Cells

Pluripotent stem cells are stem cells that have pluripotency, which is the ability to differentiate into any of three germ layers (endoderm, mesoderm, and ectoderm), and that are capable of self-replication. Pluripotent stem cells are not particularly limited. Examples include embryonic stem (ES) cells, cloned embryo-derived embryonic stem (ntES) cells obtained by nuclear transplantation, multipotent germ stem cells ("mGS cells"), embryonic germ cells ("EG cells"), induced pluripotent stem (iPS) cells, etc. Moreover, the organism from which pluripotent stem cells are derived is not particularly limited. Examples include mammals, such as humans, monkeys, mice, rats, guinea pigs, rabbits, cows, pigs, dogs, horses, cats, goats, and sheep. Of these, pluripotent stem cells derived from a human are preferable. Usable pluripotent stem cells include commercially available pluripotent stem cells, those subdivided from predetermined organizations, and those produced by a known method. ES cells and iPS cells can be preferably used as pluripotent stem cells.

ES cells can be produced by a known method. Usable ES cells may be, for example, those produced using fertilized eggs obtained by in-vitro fertilization as materials, other than those produced using fertilized eggs obtained from mothers as materials.

iPS cells can be produced by a known method, for example, introduction of reprogramming factors into any somatic cells. Examples of reprogramming factors include genes, such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1; and gene products. These reprogramming factors can be used singly or in combination of two or more. Examples of combinations of reprogramming factors include those described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612; Huangfu D et al., Nat. Biotechnol., 26:795-797 (2008); Shi Y et al., Cell Stem Cell, 2: 525-528 (2008); Eminli S et al., Stem Cells. 26: 2467-2474 (2008); Huangfu D et al., Nat. Biotechnol. 26: 1269-1275 (2008); Shi Y et al., Cell Stem Cell, 3: 568-574 (2008); Zhao Y et al., Cell Stem Cell, 3:475-479 (2008); Marson A, Cell Stem Cell, 3:132-135 (2008); Feng B et al., Nat. Cell Biol. 11:197-203 (2009); Judson RL et al., Nat. Biotechnol., 27:459-461 (2009); Lyssiotis CA et al., Proc Natl Acad Sci U S A.106: 8912-8917 (2009); Kim JB et al., Nature. 461: 649-643 (2009); Ichida JK et al., Cell Stem Cell. 5: 491-503 (2009); Heng JC et al., Cell Stem Cell. 6: 167-174 (2010); Han J et al., Nature. 463: 1096-1100 (2010); Mali P et al., Stem Cells. 28: 713-720 (2010); and Maekawa M et al., Nature. 474: 225-229(2011).

The above somatic cells are not particularly limited. Examples include fetal somatic cells, neonatal somatic cells, and matured healthy and morbid somatic cells; and also include primary culture cells, passage cells, and established cells. Specific examples of the somatic cells include (1) tissue stem cells (somatic stem cells), such as neural stem cells, hematopoietic stem cells, mesenchymal stem cells, and dental-pulp stem cells; (2) tissue precursor cells; and (3) differentiated cells, such as blood cells (peripheral blood cells, cord blood cells, etc.), lymphocytes, epithelial cells, endothelial cells, muscle cells, fibroblasts (skin cells etc.), hair cells, liver cells, gastric mucosal cells, intestinal cells, splenic cells, pancreatic cells (pancreatic exocrine cells etc.), brain cells, lung cells, renal cells, and adipocytes.

### Pancreatic Progenitor Cells

The pancreatic progenitor cells of the present invention refer to cells that subsequently differentiate into islet cells. The pancreatic progenitor cells can be identified, for example, based on whether cells are positive to PDX1 (pancreas duodenal homeobox gene 1) (and positive to SOX9).

Further, the pancreatic progenitor cells of the present invention can also be identified based on whether cells are negative to NKX6.1, NGN3 (Nerurogenin 3), etc.; however, the pancreatic progenitor cells of the present invention may be positive to NKX6.1 and/or NGN3.

In addition, markers, such as HNF6, HLXB9, PAX4, and/or NKX2-2, can also be used as indicators for the pancreatic progenitor cells.

For example, the pancreatic progenitor cells of the present invention include cells positive to markers, such as PDX1, HNF6, and HLXB9, or cells positive to markers, such as NKX6.1, NGN3, PAX4, and NKX2-2.

The pancreatic progenitor cells of the present invention are preferably PDX1-positive, and more preferably PDX1-positive and SOX9-positive.

The pancreatic progenitor cells of the present invention are particularly preferably PDX1-positive, SOX9-positive, and NKX6.1-negative and/or NGN3-negative.

In another embodiment, the pancreatic progenitor cells of the present invention are particularly preferably PDX1-positive, SOX9-positive, and NKX6.1-positive and/or NGN3-positive.

### Islet Cells

The pancreatic islet (Langerhans island) cells of the present invention include at least one member of glucagon-secreting α-cells, insulin-secreting β-cells, and somatostatin-secreting δ-cells; and preferably include at least β-cells. That the islet cells include α-cells, β-cells, and δ-cells can be confirmed, for example, by immunostaining using antibodies against glucagon, insulin or C-peptide, and somatostatin, respectively. β-cells can also be detected by immunostaining using an antibody against C-peptide. β-cells can also be detected by dithizone staining. The islet cells may further include F-cells secreting pancreatic polypeptide, and pancreatic islet progenitor cells.

### Method for Culturing Pancreatic Progenitor Cells (Step A)

The method for culturing pancreatic progenitor cells derived from pluripotent stem cells according to the present invention comprises the step of three-dimensionally culturing pancreatic progenitor cells derived from pluripotent stem cells in a medium containing (1) a factor belonging to the epidermal growth factor (EGF) family and/or a factor belonging to the fibroblast growth factor (FGF) family (hereinafter also referred to as the component (1)), and (2) Wnt agonist (hereinafter also referred to as the component (2)).

The pancreatic progenitor cells used in the culture method of the present invention are derived from pluripotent stem cells; that is, they are cells differentiated from pluripotent stem cells.

The medium used in the culture method of the present invention is a medium used for the culture of animal cells, which is used as a basal medium, and containing at least (1) a factor belonging to the FGF family and/or a factor belonging to the EGF family, and (2) Wnt agonist. The basal medium is not particularly limited, as long as it can be used for the culture of animal cells. Examples include IMDM medium, Medium 199 medium, and Eagle's Minimum Essential Medium (EMEM) medium, αMEM medium, Doulbecco's modified Eagle's Medium (DMEM) medium, Ham's F12 medium, RPMI 1640 medium, Fischer's medium, MCDB 131 medium, and their mixed media. Thus, the combined use of the component (1) and the component (2) makes it possible to efficiently proliferate pancreatic progenitor cells.

The factor belonging to the EGF family is not particularly limited, as long as it can bind to an EGF receptor and increase its activity. The factor belonging to the EGF family is preferably a factor binding to ErbB1, which is an EGF receptor. Examples include EGF, transforming growth factor-α (TGF-α), amphiregulin, heparin-binding EGF-like growth factor, schwannoma-derived growth factor, betacellulin, and poxvirus growth factor; and more preferably EGF.

The epidermal growth factor (EGF) is a polypeptide consisting of 53 amino acids and promoting the proliferation of various epidermal cells and fibroblasts, and has three intramolecular disulfide bonds. The epidermal growth factor binds to an epidermal growth factor receptor. The epidermal growth factor is also referred to as several Japanese expressions, such as an epidermal proliferation factor, an epidermal cell growth factor, a skin growth factor, and an epidermoid growth factor. The epidermal growth factor of the present invention widely includes naturally occurring epidermal growth factor variants, as long as they have natural activity. The epidermal growth factor can be a commercial product or can be produced by a known method.

As the factor belonging to the FGF family, 22 types of FGFs are present in humans and mice. FGF is also referred to as a fibroblast growth factor and a heparin-binding growth factor. Examples of the factor belonging to the FGF family include acidic fibroblast growth factors (aFGF, FGF-1), basic fibroblast growth factors (bFGF, FGF-2), FGF-3, keratinocyte growth factors (KGF, FGF-7), FGF-10, and FGF-22; preferably factors binding to FGFR2IIIb, which is an FGF receptor; and more preferably FGF-3, FGF-7, FGF-10, and FGF-22.

The Wnt agonist is a substance that activates Wnt signal transduction and that activates TCF/LEF-mediated transfer in cells. Examples include substances inducing activation upon binding to Frizzled receptors, intracellular β-catenin degradation inhibitors, TCF/LEF activators, and the like. Specific examples include factors belonging to the Wnt family (e.g., proteins belonging to the Wnt family, and low-molecular-weight compounds having the same action as that of the Wnt family), factors belonging to the R-spondin family (e.g., proteins belonging to the R-spondin family (R-spondins 1 to 4 etc.), and low-molecular-weight compounds having the same action as that of the R-spondin family), norrin, and GSK inhibitors; preferably factors belonging to the R-spondin family and/or GSK inhibitors; and more preferably proteins belonging to the R-spondin family and/or GSK inhibitors.

The protein belonging to the Wnt family is not particularly limited. Examples include Wnt-1/Int-1, Wnt-2/Irp, Wnt-2b/13, Wnt-3/Int-4, Wnt-3a, Wnt-4, Wnt-5a, Wnt-5b, Wnt-6, Wnt-7a, Wnt-7b, Wnt-8a/8d, Wnt-8b, Wnt-9a/14, Wnt-9b/14b/15, Wnt-10a, Wnt-10b/12, Wnt-11, and Wnt-16; and particularly Wnt-3a.

The GSK inhibitor is not particularly limited, as long as it is a factor that inhibits GSK-3β (Glycogen Synthase Kinase 3β). Examples include CHIR99021, SB216763, SB415286, A1070722, BIO, BIO-acetoxime, Indirubin-3'-oxime, NSC 693868, TC-G 24, TCS 2002, TWS 119, siRNA, lithium, and kenpaullone; and preferably CHIR99021.

The protein belonging to the R-spondin family is preferably R-spondin 1. R-spondin 1 belongs to the RSPO (RSPO1-4) family of Wnt modulators, and is a secreted protein that regulates Wnt/β-catenin signal transduction. The R-spondin 1 of the present invention widely includes naturally occurring R-spondin 1 variants, as long as they have natural activity. R-spondin 1 can be a commercial product or can be produced by a known method.

The medium contains at least two of a factor belonging to the EGF family, a factor belonging to the FGF family, a factor belonging to the R-spondin family (e.g., a protein or a low-molecular-weight compound having the same action as that of the R-spondin family), and a GSK inhibitor. The medium may contain 3 or more, or 4 or more, of a factor belonging to the EGF family, a factor belonging to the FGF family, a factor belonging to the R-spondin family (e.g., a protein or a low-molecular-weight compound having the same action as that of the R-spondin family), and a GSK inhibitor.

The concentration of the component (1) in the medium is not particularly limited, as long as the pancreatic progenitor cells can be proliferated. For example, the concentration of the component (1) is preferably 1 to 1000 ng/mL, and more preferably 20 to 100 ng/mL. Moreover, the concentration of the component (2) in the medium is not particularly limited, as long as the pancreatic progenitor cells can be proliferated. For example, the concentration of the component (2) is preferably 10 to 2000 ng/mL or 0.1 to 50 µM, and more preferably 200 to 1000 ng/mL or 1 to 10 µM. When the medium contains CHIR99021 as a GSK inhibitor, the concentration of CHIR99021 is preferably 1 to 20 µM, and more preferably 3 to 10 µM.

The concentration of the epidermal growth factor in the medium is not particularly limited, as long as the pancreatic progenitor cells can be proliferated. For example, the concentration of the epidermal growth factor is preferably 1 to 1000 ng/mL, and more preferably 20 to 100 ng/mL. Moreover, the concentration of R-spondin-1 in the medium is not particularly limited, as long as the pancreatic progenitor cells can be proliferated. For example, the concentration of R-spondin-1 is preferably 10 to 2000 ng/mL, and more preferably 200 to 1000 ng/mL.

The medium used in the present invention preferably further contains at least one member selected from the group consisting of a Sonic Hedgehog signal inhibitor, a TGF-β receptor inhibitor, and retinoic acid.

The Sonic Hedgehog signal inhibitor is not particularly limited, as long as it is a factor that can inhibit Sonic Hedgehog signals. Specific examples include SANT-1, Jervine, Cyclopamine-KAAD, and the like.

The TGF-β receptor inhibitor is not particularly limited, as long as it is a factor that can inhibit the function of TGF-β receptors. Specific examples include LDN193189, D4476, LY2157299, LY364947, SB525334, SB431542, SD208, and the like. Further, BMP signal inhibitors, such as dorsomorphin and Noggin, can also be used in place of the TGF-β receptor inhibitor. When long-term culture is performed by repeating passages, it is desirable to add a TGF-β receptor inhibitor and/or a BMP signal inhibitor to the medium.

All-trans retinoic acid is particularly preferably used as the retinoic acid.

Further, the medium used in the present invention may contain a ROCK (Rho-associated coiled-coil forming kinase/Rhobinding kinase) inhibitor. It is desirable that the ROCK inhibitor be added to the medium only 1 to 2 days after passage.

The ROCK inhibitor is not particularly limited, as long as it is a factor that can inhibit the function of ROCK. Specific examples include Y-27632, Fasudil (or HA1077), H-1152, Wf-536, Y-30141, and the like.

The medium used in the present invention may contain serum or may be serum-free; a serum-free medium is preferable.

The medium used in the present invention may further contain serum replacements (e.g., albumin, transferrin, Knockout Serum Replacement (KSR), fatty acid, insulin, collagen precursor, minor element, 2-mercaptoethanol, 3'-thiolglycerol, and ITS-supplement). Serum replacements can be used singly or in combination of two or more.

The medium used in the present invention may further contain one or more substances of B27-supplement, N2-supplement, lipids, glucose, amino acids (non-essential amino acids etc.), L-glutamine, Glutamax (Thermo Fisher Scientific), vitamins, growth factors, cytokines, antibiotics, antioxidants, pyruvic acid, buffers, inorganic salts, etc.

As the medium used in the present invention, it is desirable to use a chemically-defined medium that does not contain materials with unknown components, such as serum, because the difference in medium among lots can be reduced, and pancreatic progenitor cells with stable quality can be prepared.

The pH of the medium used in the present invention is generally 7.0 to 7.8, and preferably 7.2 to 7.6. In order to prevent contamination before use, the medium is preferably sterilized by a method such as filtration, UV irradiation, heat sterilization, or radiation.

The medium used in the present invention can be prepared in a solution form, a dried form, or a concentrated form (e.g., 2x to 1000x). The medium in a concentrated form can be used after being suitably diluted to a suitable concentration. The liquid used to dilute the medium in a dried form or a concentrated form is suitably selected from water, a buffer solution, a saline solution, etc.

The pancreatic progenitor cells of the present invention are three-dimensionally cultured. The three-dimensional culture mentioned herein is a culture method that performs culture with thickness in the longitudinal direction, unlike two-dimensional culture, which performs monolayer culture. It is possible to efficiently proliferate the pancreatic progenitor cells by performing such three-dimensional culture. As the three-dimensional culture method, known methods can be widely used without limitation, as long as the pancreatic progenitor cells can be proliferated. In particular, suspension culture using cell aggregates of the pancreatic progenitor cells is preferable.

The culture method that forms cell aggregates of the pancreatic progenitor cells is explained below.

First, the pancreatic progenitor cells are dissociated into dispersed cells (single cells or several cell masses). Dissociation into dispersed cells can be performed by treatment using enzymes, such as trypsin and collagenase, or a chelating agent, such as EDTA, or by mechanical operation, such as pipetting. The pancreatic progenitor cells dissociated into dispersed cells are suspended in a medium, and seeded in a culture container at a cell concentration of preferably 10 to 10000 cells/well, and more preferably 300 to 3000 cells/well. Then, the cells are allowed to stand in this state for a certain period of time (e.g., 12 to 36 hours), thereby forming aggregates. The size (diameter) of the aggregate is generally about 50 to 500 µm, and preferably about 100 to 200 µm. The number of cells per aggregate is generally about 100 to 5000, and preferably about 500 to 2000.

In the culture method that forms cell aggregates of the pancreatic progenitor cells, a culture container having culture wells with a capacity of, for example, 0.001 to 10 µL/well, 0.001 to 1 µL/well, 0.005 to 0.5 µL/well, 0.01 to 0.5 µL/well, or 0.01 to 0.1 µL/well, can be used. Moreover, it is preferable to use culture containers having culture wells with a shape that allows cells to sink to the bottom and to easily form aggregates, for example, hemispherical culture wells having a bottom portion expanded toward the bottom, or cylindrical culture wells having a hemispherical bottom portion. The diameter of the culture well of the culture container is, for example, 200 to 800 µm or 400 to 800 µm. Moreover, the depth of such a culture well is, for example, 400 to 1000 µm or 400 to 800 µm. Many pancreatic progenitor cells can be obtained using a multi-well culture container having multiple wells with the above shape.

As the culture container for forming cell aggregates of the pancreatic progenitor cells, in order to perform non-adhesion culture, the culture container surface may be subjected to cell non-adhesion treatment (for example, an culture container made of plastic (e.g., polystyrene) that has been subjected to cell non-adhesion treatment), but is preferably made of a material that allows culture of cells in a non-adhesion state. Such a material is preferably a hydrophilic material having a three-dimensional structure without cytotoxicity, and more preferably a transparent material so as to facilitate observation of the culturing state. Moreover, hydrogels comprising a hydrophilic polymer used for non-adhesion treatment of cells are also preferable.

Examples of the material used to produce hydrogels include physical or chemical crosslinked products of synthetic polymers, such as polyvinyl alcohol, polyvinyl pyrrolidone, polyethylene glycol, poly-2-hydroxyethyl methacrylate, poly-2-hydroxyethyl acrylate, polyacrylamide, polyacrylic acid, and polymethacrylic acid; crosslinked products of the above synthetic polymers obtained by radiation; crosslinked products of copolymers of monomers constituting the above polymers; and other various synthetic polymer materials that can form hydrogels. Moreover, it is also possible to use natural polymers, including polysaccharides (e.g., agarose, alginic acid, dextran, and cellulose) and derivatives thereof; and crosslinked products of proteins, such as gelatin and albumin, and derivatives thereof. Of these, agarose gel is preferable as the material used for producing hydrogels.

Examples of the material used for treatment so that the cells can be cultured in a non-adhesion state include materials used for the production of hydrogels mentioned above, polymer materials comprising 2-methacryloyloxyethyl phosphorylcholine (MPC) as a main component, and the like.

The culture conditions of the culture method of the present invention may be the same as conditions for general cell culture. The culture temperature is preferably 35 to 39°C, and more preferably 37°C. The O₂ concentration is generally about 5 to 20%. The CO₂ concentration is generally about 1 to 10%, and preferably about 5%. Such culture can be performed using a known CO₂ incubator.

The culture time is not particularly limited. For example, the culture time is preferably 3 to 10 days, and more preferably 5 to 7 days. Moreover, it is preferable to exchange the medium at intervals of one to three days during culture.

The culture method of the present invention is preferably carried out in the absence of feeder cells. Since the culture method of the present invention allows culture in the absence of feeder cells, unknown components are not mixed, and pancreatic progenitor cells having uniform properties can be stably proliferated.

The culture method of the present invention can efficiently proliferate pancreatic progenitor cells with proliferation potential higher than that of cells differentiated into islet cells and other functional cells, such as liver cells, nerve cells, and pancreatic exocrine cells, and is thus suitably used for the purification of pancreatic progenitor cells.

### Subculture (Step B)

In a preferable embodiment of the culture method of the present invention, the method further comprises the step of subculturing the pancreatic progenitor cells obtained in step A.

Subculture can be performed by collecting aggregates of the pancreatic progenitor cells cultured by the above method, dissociating the aggregates into dispersed cells, seeding the dispersed cells in a new medium, and culturing them. Dissociation into dispersed cells, cell seeding, medium, culture method, etc., are the same as those described above.

The number of times of passage is, for example, 1 to 10, 1 to 5, or 2 or 3; and preferably 3 or more. In the present specification, the first culture is denoted by the 0th passage.

In the culture method of the present invention, the proliferation potential of the pancreatic progenitor cells is maintained even when they are subcultured; thus, large amounts of pancreatic progenitor cells can be prepared. Furthermore, as the number of times of passage increases, the purification of pancreatic progenitor cells is also improved.

### Step of Preparing iPS Cells (Step C)

The culture method of the present invention may further comprise the step of preparing iPS cells.

Examples of the method for preparing iPS cells include the methods described in the above "Pluripotent Stem Cells" section. The iPS cells prepared in this step can be differentiated into pancreatic progenitor cells, and the pancreatic progenitor cells can be used in the culture of step A.

In the present invention, when pluripotent stem cells other than iPS cells are used, the other pluripotent stem cells can be prepared in this step, in place of iPS cells.

### Step of Inducing Differentiation into Pancreatic Progenitor Cells (Step D)

The culture method of the present invention may further comprise the step of inducing the differentiation of pluripotent stem cells into pancreatic progenitor cells.

The pancreatic progenitor cells differentiated in this step can be used in the culture of step A.

In the step of differentiating pluripotent stem cells into pancreatic progenitor cells, the composition of the culture solution may be changed with time so as to imitate the process of in-vivo pancreas development. Moreover, the differentiation-inducing step can be performed by suspension culture using cell aggregates described above or adhesion culture.

As such a method, for example, the methods disclosed in the following documents, and suitably modified versions of these methods can be used. According to the method disclosed in Reference Document 2, pluripotent stem cells can be differentiated into pancreatic progenitor cells by carrying out Stages 1 to 4.
Reference Document 1: Rezania A, Bruin JE, Riedel MJ, Mojibian M, Asadi A, Xu J, Gauvin R, Narayan K, Karanu F, O'Neil JJ, Ao Z, Warnock GL, Kieffer TJ. Maturation of human embryonic stem cell-derived pancreatic progenitors into functional islets capable of treating pre-existing diabetes in mice. Diabetes 2012; 61: 2016-2029.
Reference Document 2: Rezania A, Bruin JE, Arora P, Rubin A, Batushansky I, Asadi A, O'Dwyer S, Quiskamp N, Mojibian M, Albrecht T, Yang YH, Johnson JD, Kieffer TJ. Reversal of diabetes with insulin-producing cells derived in vitro from human pluripotent stem cells. Nat Biotechnol 2014; 32: 1121-1133.
Reference Document 3 : Hrvatin S, O'Donnell CW, Deng F, Millman JR, Pagliuca FW, DiIorio P, Rezania A, Gifford DK, Melton DA. Differentiated human stem cells resemble fetal, not adult, β cells. Proc Natl Acad Sci U S A. 2014; 111: 3038-3043
Reference Document 4: Pagliuca FW, Millman JR, Gurtler M, Segel M, Van Dervort A, Ryu JH, Peterson QP, Greiner D, Melton DA. Generation of functional human pancreatic β cells in vitro. Cell. 2014; 159: 428-439.

For example, it is preferable to add Activin A and Wnt3a in the initial stage, and it is also preferable to subsequently add retinoic acid and hedgehog signal inhibitors (e.g., SANT-1 and Cyclopamine-KAAD), fibroblast growth factors, etc.

Moreover, in the process of differentiation, in order to imitate the process of in-vivo pancreas development to obtain pancreatic progenitor cells, substances that maintain undifferentiated properties and promote proliferation, substances that suppress proliferation and promote differentiation, proteins expressed in the pancreas in vivo, etc., may be added at an appropriate time. Examples of such substances include GSK-3β (Glycogen Synthase Kinase 3β) inhibitors (e.g., CHIR99021), ALK inhibitors (e.g., SB431542), Notch signal inhibitors (e.g., DAPT), TGFβ inhibitors (e.g., LDN193189), AMPK and BMP signal inhibitors (e.g., Dorsomorphin), PKC activators (e.g., Pdbu), insulin-like growth factor-1, epidermal growth factors, hepatocyte growth factors, glucagon-like peptide-1, commercially available supplements, and the like.

### Step of Inducing Differentiation into Islet Cells (Step E)

The method for producing islet cells from pancreatic progenitor cells derived from pluripotent stem cells according to the present invention comprises the step of inducing the differentiation of pancreatic progenitor cells cultured by the above method into islet cells.

In the step of differentiating the pancreatic progenitor cells into islet cells, the composition of the culture solution may be changed with time so as to imitate the process of in-vivo pancreas development. The differentiation-inducing step can be performed by suspension culture using the cell aggregates described above.

As such a method, for example, the methods disclosed in Reference Documents 1 to 4 mentioned above, and suitably modified versions of these methods can be used. According to the method disclosed in Reference Document 2, pancreatic progenitor cells can be differentiated into islet cells by carrying out Stages 5 to 7.

### Step of Freezing Pancreatic Progenitor Cells (Step F)

The method for cryopreserving pancreatic progenitor cells derived from pluripotent stem cells according to the present invention comprises the step of freezing pancreatic progenitor cells cultured by the above method.

As the pancreatic progenitor cells, cells cultured by the above culture method, and further subcultured cells can be both used. Moreover, it is desirable that the pancreatic progenitor cells to be cryopreserved be dissociated into dispersed cells. The method for dissociating the pancreatic progenitor cells into dispersed cells is the same as that described above.

The cryopreservation solution is not particularly limited. Examples include commercially available cryopreservation solutions (e.g., CELLBANKER (registered trademark) 2 (Nippon Zenyaku Kogyo Co., Ltd.), STEM-CELLBANKER (registered trademark) (Nippon Zenyaku Kogyo Co., Ltd.), and StemSure (registered trademark) Freezing Medium (Wako Pure Chemical Industries, Ltd.)), culture media to which about 5 to 20 volume% of dimethylsulfoxide is added (e.g., the medium used in the above culture method), and the like.

Cryopreservation can be performed by freezing generally at about -70 to -196°C, and preferably at -100°C or less. For long-term storage, storage can be performed in liquid nitrogen, or in the vapor phase above the liquid nitrogen, in a liquid nitrogen cell preservation container.

The cryopreserved cells can be thawed by rapidly warming in a water bath generally at about 20 to 40°C, and preferably at about 35 to 38°C.

The pancreatic progenitor cells proliferated by the culture method of the present invention maintains, after cryopreservation, a proliferation potential equivalent to that before cryopreservation. Therefore, this technique is expected to be applied to cell banks of pancreatic progenitor cells.

### Examples

Examples are provided below in order to explain the present invention in more detail. However, the present invention is not limited to these Examples.

In the following Examples, when the name of the iPS cell line is not given, experimental results using the 253G1 line are shown.

### Preparation of Agarose Microwells

Agarose microwells were prepared using a 3D Petri Dish (produced by Microtissues, Inc.) with reference to the protocol provided by the manufacturer (http://www.funakoshi.co.jp/contents/5556). A mold for a 256-well/gel-plate having a well diameter of 400 µm was used. Specifically, the agarose microwells were prepared by the following procedure.

First, a warmed agarose solution (Lonza agarose, 2.5% agarose/physiological saline) was poured into the mold. Next, the mold was cooled to room temperature, and after gelation of the agarose, the agarose microwells were removed from the mold. The agarose microwells were transferred to a 12-well polystyrene plate for cell culture, and a medium (DMEM/F12) was added in the vicinity of the agarose microwells to immerse the agarose microwell plate therein. The plate was placed in an incubator (37°C, 5% CO₂) for one night or more to equilibrate the agarose microwell plate with the medium in its vicinity. Thus, agarose microwells having 256 wells, each of which had a cylinder part (diameter: 400 µm) and a hemispherical bottom, were obtained. The above operation was performed under aseptic conditions in a clean bench.

### Formation of Aggregates, and Differentiation Induction into Pancreatic Progenitor Cells

iPS cells (253G1, obtained from Riken Cell Bank) were cultured in E8 medium (Thermo Fisher Scientific) for 3 to 4 days using a culture container coated with Geltex (Thermo Fisher Scientific). After treatment using TrypLE (Thermo Fisher Scientific) under 70 to 80% confluent conditions, the cells were collected as single cells. The cells were suspended in E8 medium containing 10 µM Y-27632 (ROCK inhibitor, Wako Pure Chemical Industries, Ltd.), and seeded at 2500 cells/well on average in the 256-well agarose microwell plate, which was prepared as described above and placed in one well of the 12-well polystyrene plate.

After the agarose microwell plate was left to stand for 10 minutes to precipitate the cells in the bottom, a medium (E8 medium + ROCK inhibitor) was added in the vicinity of the agarose microwell plate to immerse the plate in the medium. The cells were cultured at 37°C with 5% CO₂ for 24 hours (5% CO₂, 37°C) to aggregate the cells, and then cultured for a certain period of time to induce their differentiation. Specifically, medium replacement was performed in such a manner that the medium was sucked out every day, and a new medium was added, as described below. In addition, the medium composition was changed on predetermined days. The medium composition and the number of days of culture in each medium were determined according to the description of A Rezania et al. Reversal of diabetes with insulin producing cells derived in vitro from human pluripotent stem cells. Nat Biotechnol. 2014 Nov; 32 (11): 1121-33.

### First Stage (3 days)

MCDB131 (Thermo Fisher Scientific) + 1.5 g/L NaHCO₃ (Nacalai Tesque, Inc.) + 0.5% fat-free BSA (Wako Pure Chemical Industries, Ltd.) + 2 mM GlutaMax (Thermo Fisher Scientific) + 10 mM D-Glucose (Nacalai Tesque, Inc.) + 3 µM CHIR99021 (Tocris Bioscience) + 100 ng/mL Activin A (R&D Systems) (CHIR99021 was added to the medium only on the first day).

### Second Stage (2 days)

MCDB131 + 1.5 g/L NaHCO₃ + 0.5% fat-free BSA + 2 mM GlutaMax + 10 mM D-glucose + 50 ng/mL fibroblast growth factor 7 (FGF-7, PeproTech) + 0.25 mM ascorbic acid (Sigma-Aldrich)

### Third Stage (2 days)

MCDB131 + 1.5 g/L NaHCO₃ + 0.5% fat-free bovine serum albumin (fat-free BSA) + 1/200 ITS supplement (Thermo Fisher Scientific) + 2 mM GlutaMax + 20 mM D-glucose + 50 ng/mL FGF-7 + 0.25 µM SANT-1 (Wako Pure Chemical Industries, Ltd.) + 0.1 µM LDN193189 (Wako Pure Chemical Industries, Ltd.) + 1 µM retinoic acid (Sigma-Aldrich) + 0.2 µM TBP (PKC activator; Catalog No. 565740; EMD Chemicals Inc.) + 0.25 mM ascorbic acid

### Fourth Stage (2 days)

MCDB131 + 1.5 g/L NaHCO₃ + 0.5% fat-free BSA + 1/200 ITS supplement + 2 mM GlutaMax + 20 mM D-glucose + 50 ng/mL FGF-7 + 0.25 µM SANT-1 + 0.2 µM LDN193189 + 0.1 µM retinoic acid + 0.1 µM TBP + 0.25 mM ascorbic acid
TBP: ((2S,5S)-(E,E)-8-(5-(4-(trifluoromethyl)phenyl)-2,4-pentadienoylamino)benzolactam

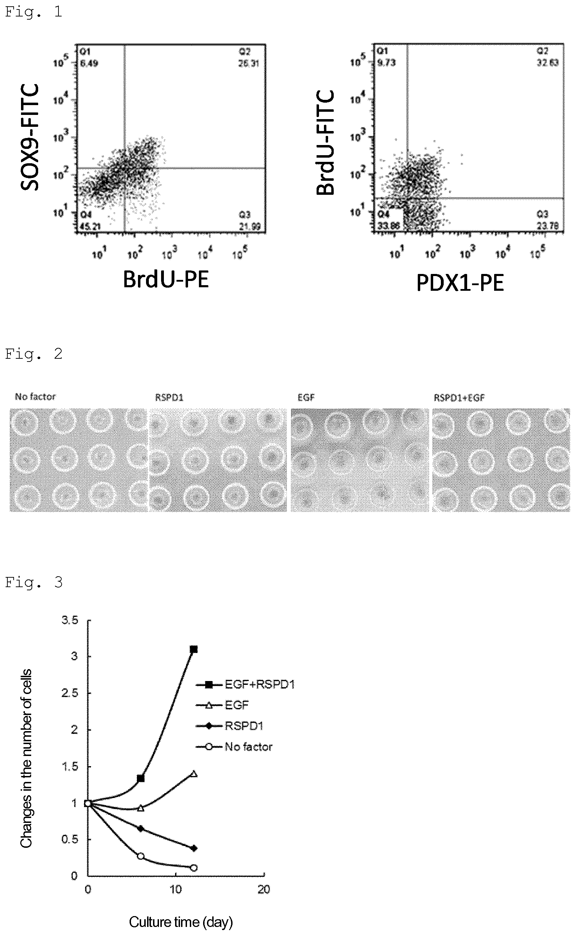

Fig. 1 shows the results of flow cytometry analysis of cells obtained by culture of the above first to fourth stages. In Fig. 1, SOX9 is a pancreatic progenitor cell marker, BrdU is a cell proliferation marker (a nucleic acid analog for marking the nuclei of cells with proliferation potential), and PDX1 is a pancreatic progenitor cell and islet cell marker. BrdU was added to the culture solution, and culture was performed for 24 hours, followed by staining and flow cytometry analysis. Goat anti-PDX1 antibody (R&D systems), rabbit anti-SOX9 antibody (Merck Millipore), and mouse anti-BrdU antibody (Dako) were used as primary antibodies. FITC-labeled anti-mouse IgG antibody (Thermo Fisher Scientific), PE-labeled anti-goat IgG antibody (Thermo Fisher Scientific), FITC-labeled anti-rabbit IgG antibody (BD Biosciences), and PE-labeled anti-mouse IgG antibody (BD Biosciences) were used as secondary antibodies. For measurement, Guava (registered trademark) easyCyte (Merck Millipore) was used.

Fig. 1 indicates that about 60% of the cells differentiated into PDX1-positive pancreatic cells, and that about 30% of SOX9/BrdU-positive pancreatic progenitor cells under proliferation were contained. As a result, a non-homogeneous cell population was obtained. It was assumed that undifferentiated cells, pancreatic progenitor cells (SOX9- and PDX1-positive, and NKX6.1-negative cells), and cells matured into endocrine cells were contained.

### Amplification of Pancreatic Progenitor Cells

The cell aggregates obtained by culture of the above first to fourth stages were dispersed into single cells using a cell dispersion enzyme solution TrypLE (Thermo Fisher Scientific). The cells were suspended in the following medium containing 10 µM Y-27632 (ROCK inhibitor, Wako Pure Chemical Industries, Ltd.), and seeded at 1000 cells/well (1000 x 256 = 2.56 x 10⁵/plate) in the 256-well agarose microwell plate placed on a well of the 12-well plate. After the agarose microwell plate was left to stand for 10 minutes to precipitate the cells in the bottom, the following medium was added in the vicinity of the agarose microwell plate to immerse the plate in the medium. Thereafter, culture was performed for 6 days at 37°C with 5% CO₂. Medium replacement was performed every other day.
MCDB131 + 1.5 g/L NaHCO₃ + 0.5% fat-free BSA + 1/200 ITS supplement + 2 mM GlutaMax + 20 mM D-glucose + 50 ng/mL epidermal growth factor (EGF, Wako Pure Chemical Industries, Ltd.) + 200 ng/mL r-spondin 1 (RSPD1, R&D Systems) + 0.25 µM SANT-1 (Wako Pure Chemical Industries, Ltd.) + 0.2 µM LDN193189 (Wako Pure Chemical Industries, Ltd.) + 0.1 µM retinoic acid (Sigma-Aldrich)

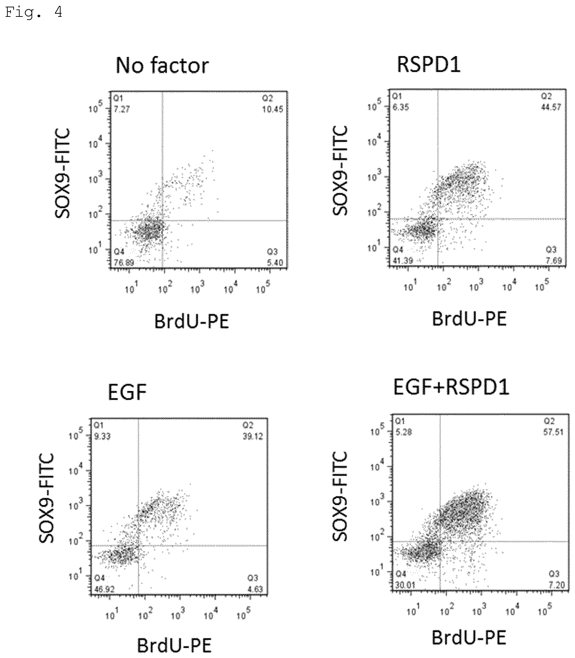

Figs. 2 to 4 show the results. In Fig. 3, the cells were once passaged after 6 days. In Fig. 4, BrdU was added to the culture solution, and culture was performed for 24 hours, followed by staining and flow cytometry analysis. Cells proliferated during 24-hour culture are labelled with BrdU. In Fig. 4, SOX9 is a pancreatic progenitor cell marker. Figs. 2 and 3 indicate that the cells were proliferated well when r-spondin 1 and EGF were co-added. Further, Fig. 4 indicates that the ratio of SOX9/BrdU-positive cells was highest when r-spondin 1 and EGF were co-added. The rate of SOX9- and PDX1-positive pancreatic progenitor cells was increased (from 26.3% to 57.5%) as compared with the rate before culture, and screening into pancreatic progenitor cells progressed.

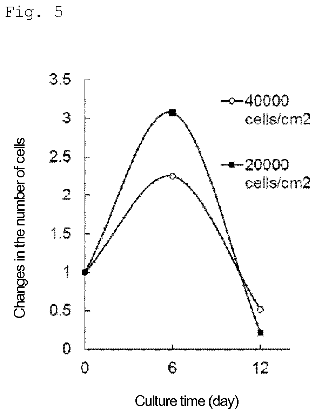

The cells obtained by culture of the above first to fourth stages were attached to a plate substrate at a density of 20000 or 40000 cells/cm² on a 6-well plate coated with Geltrex, and adhesion culture was performed by the same culture method as in "Amplification of Pancreatic Progenitor Cells" described above. The cells were cultured for 12 days at 37°C with 5% CO₂. The cells were passaged 6 days after culture. Medium replacement was performed every other day. Fig. 5 shows the results. Fig. 5 indicates that when adhesion culture was performed, many cells differentiated into exocrine cells (no data shown), that the proliferative ability of the cells was reduced in the middle of repeating passages, and that the number of cells took a downward turn.

### Purification of Pancreatic Progenitor Cells

The cell aggregates were collected from the agarose microwell plate at intervals of six days, dispersed into single cells, and seeded in a new agarose microwell plate. The cell dispersion method, medium composition, and culture conditions were the same as those in "Amplification of Pancreatic Progenitor Cells" above.

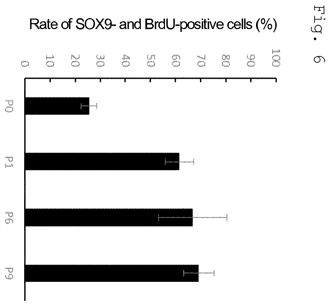

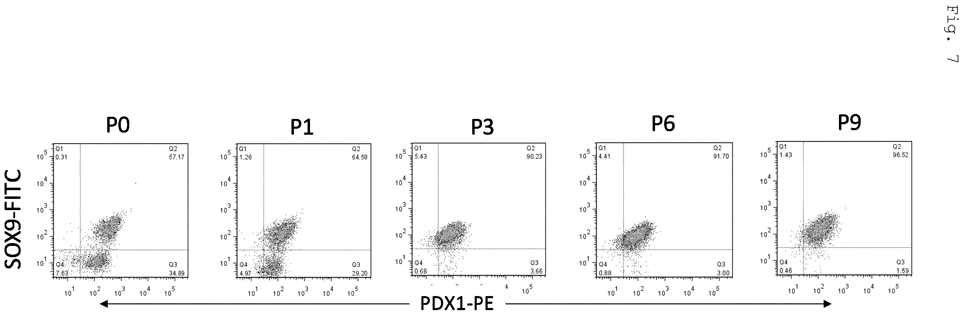

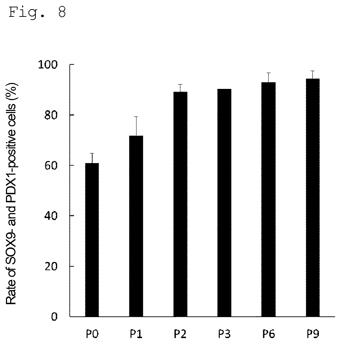

Fig. 6 shows the results of measuring the ratio of SOX9- and BrdU-positive cells in each passage. In Fig. 6, P means the number of times of passage. Fig. 6 indicates that the ratio of SOX9/BrdU-positive cells increased as passage was repeated, and increased to 60% after one passage. Figs. 7 and 8 show the results of measuring the ratio of SOX9- and PDX1-positive cells in each passage. The ratio of pancreatic progenitor cells positive to SOX9 and PDX1 increased as passage was repeated, and increased to 90% after three passages. These results suggest that the purity of progenitor cells with higher proliferation potential than that of mature cells increased.

### Long-Term Culture of Pancreatic Progenitor Cells

The cell aggregates were collected from the agarose microwell plate at intervals of six days, dispersed into single cells including SOX9- and PDX1-positive pancreatic progenitor cells, and then seeded in a new agarose microwell plate. The cell dispersion method, medium composition, and culture conditions were the same as those in "Amplification of Pancreatic Progenitor Cells" above.

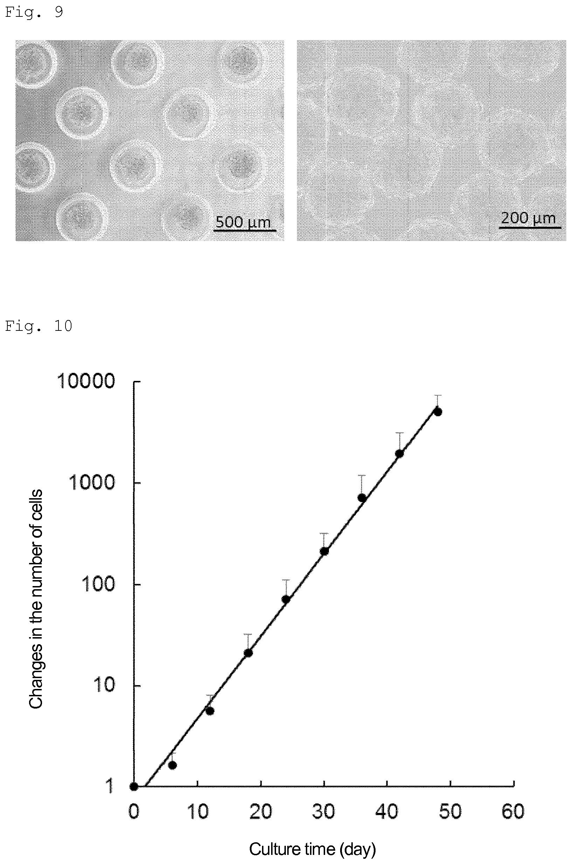

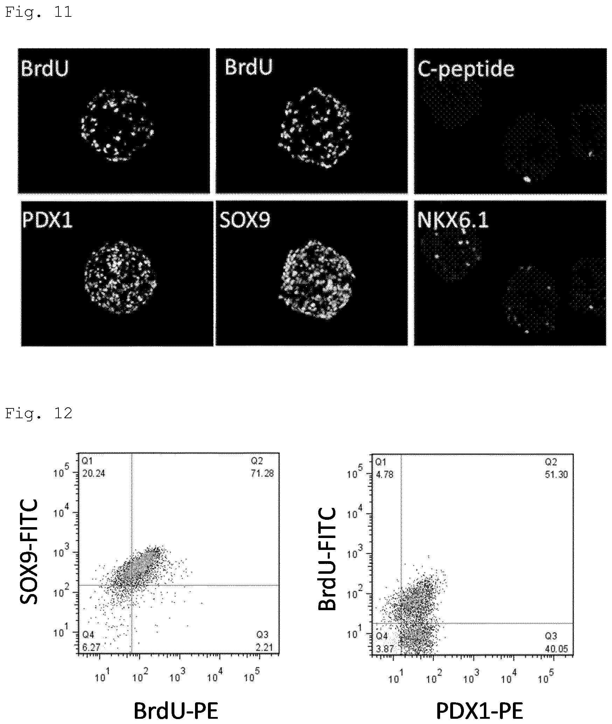

Figs. 9 to 12 show the results. The left figure of Fig. 9 shows a phase-contrast microscope image of cell aggregates cultured on the agarose microwells, and the right figure shows a phase-contrast microscope image of the cell aggregates taken from the agarose microwell plate. In Figs. 11 and 12, BrdU is a cell proliferation marker, PDX1 is a pancreatic progenitor cell and islet cell marker, SOX9 is a pancreatic progenitor cell marker, C-peptide is a β-cell marker, and NKX6.1 is an endocrine cell marker. In a cell population containing various cells, aggregates have a distorted shape; however, Fig. 9 shows that the obtained cell aggregates have a shape similar to a spherical shape, and it is thus assumed that the cells in the aggregates are uniform pancreatic progenitor cells. Fig. 10 indicates that amplification was possible for a long period of time (48 days) without reduction in cell proliferative potential, and that the number of cells increased 3 times for each passage, i.e., culture for 6 days. Figs. 11 and 12 indicate that the cells amplified for a long period of time were positive to the pancreatic progenitor markers SOX9 and PDX1. Moreover, when BrdU was added to the culture solution, and culture was performed for 24 hours, followed by staining, many BrdU-positive cells under proliferation were observed. However, there were only a few cells with progressive maturation positive to C-peptide as a β-cell marker and positive to NKX6.1 as an endocrine cell marker.

### Maturation into Endocrine Cells

The pancreatic progenitor cells amplified and cultured for six passages were seeded in an agarose well plate in the same manner as in "Amplification of Pancreatic Progenitor Cells" above. The seeding density was 3000 cells/well. The cells were matured into endocrine cells in the procedure shown in the following first to third stages. Specifically, the medium composition was changed with time, as described below. The medium was sucked out every other day and replaced with a new medium. In addition, the medium composition was changed on predetermined days. The medium composition and the number of days of culture in each medium were determined according to the description of A Rezania et al. Reversal of diabetes with insulin-producing cells derived in vitro from human pluripotent stem cells. Nat Biotechnol. 2014 Nov; 32 (11): 1121-33.

### First Stage (3 days)

MCDB131 + 1.5 g/L NaHCO₃ + 0.5% fat-free BSA + 1/200 ITS supplement + 2 mM GlutaMax + 20 mM D-glucose + 0.25 µM SANT-1 + 0.2 µM LDN193189 + 0.05 µM retinoic acid + 1 µM T3 (Thyroid hormone, triiodothyronine, Sigma-Aldrich) + 10 µM Alk5i II (activin receptor-like kinase receptors 5 inhibitor II, Enzo Life Sciences, Inc.) + 10 µg/mL heparin (Nacalai Tesque, Inc.) + 10 µM Zinc Sulfate (Sigma-Aldrich)

### Second Stage (7 days)

MCDB131 + 1.5 g/L NaHCO₃ + 0.5% fat-free BSA + 1/200 ITS supplement + 2 mM GlutaMax + 20 mM D-glucose + 0.2 µM LDN193189 + 1 µM T3 + 10 µM Alk5i II + 10 µg/mL heparin + 10 µM Zinc Sulfate + 0.1 µM GSi XX (gamma-secretase inhibitor XX, Merck Millipore) Third Stage (7 to 14 days)
MCDB131 + 1.5 g/L NaHCO₃ + 0.5% fat-free BSA + 1/200 ITS supplement + 2 mM GlutaMax + 20 mM D-glucose + 1 µM T3 + 10 µM Alk5i II + 10 µg/mL heparin + 10 µM Zinc Sulfate + 1 mM N-Cys (N-acetylcysteine, Sigma-Aldrich) + 2 µM R428 (Axl inhibitor, Selleckchem) + 10 µM Trolox (Merck Millipore)

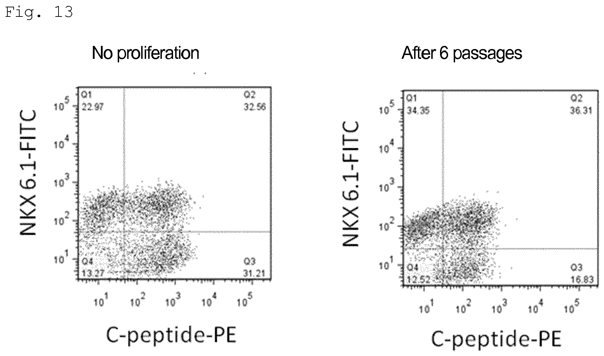

For the purpose of examining differentiation induction efficiency, C-peptide-positive pancreatic β-cells in the cell aggregates differentiated into islet cells were examined. Fig. 13 shows the results. In Fig. 13, NKX6.1 is an endocrine cell marker, and C-peptide is a β-cell marker. Fig. 13 indicates that when pancreatic progenitor cells amplified and cultured for six passages were used, the cells differentiated into C-peptide/NKX6.1-positive β cells at a ratio equivalent to that of pancreatic progenitor cells without amplification.

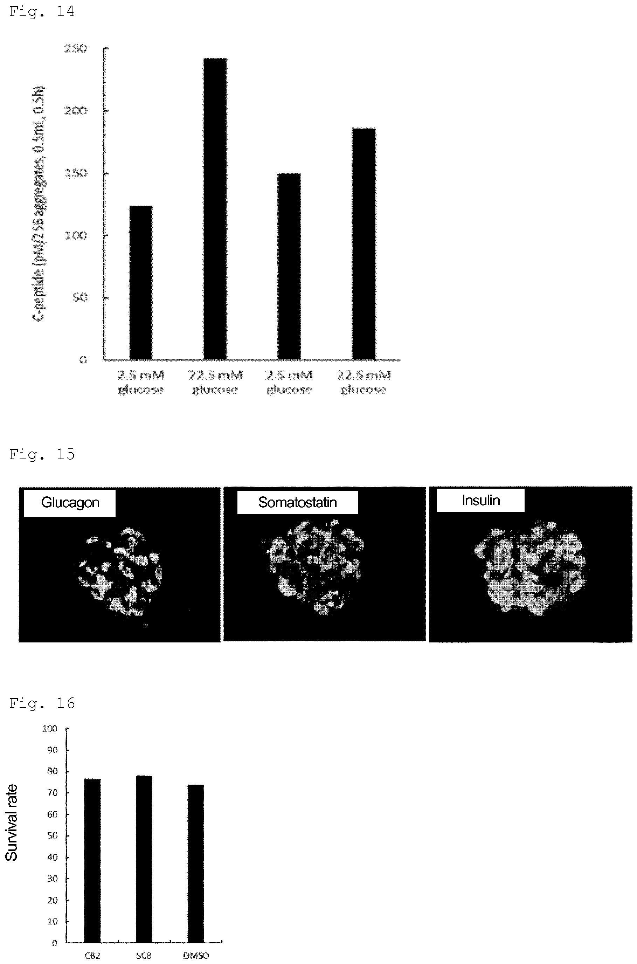

A glucose tolerance test was conducted on the cell aggregates differentiated into islet cells. Specifically, the aggregates were immersed in Krebs Ringer's solutions containing 2.5 mM, 22.5 mM, 2.5 mM, and 22.5 mM glucose for 30 minutes, and C-peptide secreted into the Krebs Ringer's solutions was quantified by an ELISA (enzyme-linked immunosorbent assay) method. Fig. 14 shows the results. Fig. 14 reveals that in the cells matured from the pancreatic progenitor cells, the C-peptide secretion amount varies depending on the glucose concentration. As a result, it was indicated that the amplified pancreatic progenitor cells were allowed to differentiate into pancreatic endocrine cells, including β-cells, and had the ability to change the insulin secretion amount in response to the glucose concentration.

Fig. 15 shows the results of immunostaining the cell aggregates differentiated into islet cells. In Fig. 15, glucagon is an α-cell marker, somatostatin is a δ-cell marker, and insulin is a β-cell marker. Fig. 15 indicates that the cell aggregates differentiated into islet cells also included glucagon-positive α-cells and somatostatin-positive δ-cells.

### Cryopreservation of Pancreatic Progenitor Cells

### - Freezing

After five passages in the same manner as in "Amplification of Pancreatic Progenitor Cells" above, the pancreatic progenitor cells were dispersed into single cells. The cells were frozen by a slow-freezing method using a commercially available cryopreservation solution (CELLBANKER 2 (Nippon Zenyaku Kogyo Co., Ltd.) or STEM-CELLBANKER (Nippon Zenyaku Kogyo Co., Ltd.)) or a solution obtained by adding 10% dimethylsulfoxide to the culture solution used for proliferation. Specifically, 5 x 10⁵ cells were suspended in 500 µL of cryopreservation solution, and injected into freezing vials. The vials were placed in a freezing container (Bicell, Nihon Freezer Co., Ltd.), and stored at -80°C overnight. In the case of long-term storage, the vials were transferred to a liquid nitrogen tank and stored therein.

### - Thawing Method

The freezing vials stored at -80°C for 1 day and in a liquid nitrogen storage tank for 6 hours were rapidly thawed by warming in a water bath at 37°C. The thawed cell suspension was added to 10 mL of culture solution (MCDB131 + 1.5 g/L NaHCO₃ + 0.5% fat-free BSA + 1/200 ITS supplement + 2 mM GlutaMax + 20 mM D-glucose). After the supernatant was removed by centrifugal separation, the cells were suspended in a medium containing 10 µM Y-27632 (MCDB131 + 1.5 g/L NaHCO₃ + 0.5% fat-free BSA + 1/200 ITS supplement + 2 mM GlutaMax + 20 mM D-glucose + 50 ng/mL EGF + 200 ng/mL r-spondin 1 + 0.25 µM SANT-1 + 0.2 µM LDN193189 + 0.1 µM retinoic acid + 10 µM Y-27632). A trypan blue stain was added thereto, and the cell survival rate after thawing was calculated.

Fig. 16 shows the results. In Fig. 16, CB2 shows the results using CELLBANKER 2 as a cryopreservation solution, SCB shows the results using STEM-CELLBANKER as a cryopreservation solution, and DMSO shows the results using a medium + 10% DMSO (self-made cryopreservation solution) as a cryopreservation solution. Fig. 16 indicates that the survival rate was 70 to 80% when any cryopreservation solution was used.

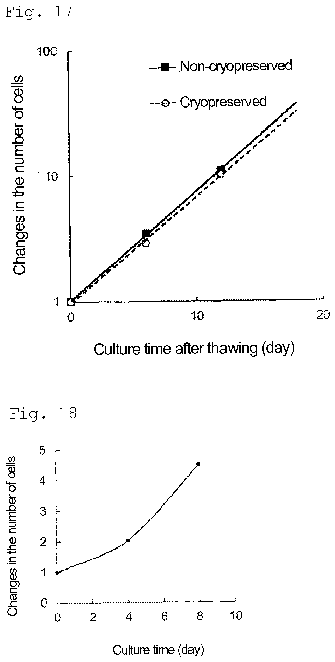

Further, Fig. 17 shows changes in the number of cells when the cells after thawing were passaged at intervals of six days by three-dimensional culture in the same manner as in "Amplification of Pancreatic Progenitor Cells" above. Fig. 17 shows the results of cells cryopreserved using CELLBANKER 2. The results shown in Fig. 17 clearly indicate that the cryopreserved cells have proliferation potential equivalent to that of non-cryopreserved cells.

### Differentiation Induction into Pancreatic Progenitor Cells Derived from Other iPS Cell Lines

454E2 line (obtained from Riken Cell Bank), RPChiPS771-2 line (ReproCELL, Inc.), and P11025 line (Takara Bio, Inc.) were used as human-derived iPS cells.

The 454E2 line was cultured in E8 medium (Thermo Fisher Scientific) for 3 to 4 days using a culture container coated with Geltex (Thermo Fisher Scientific). After treatment using TrypLE (Thermo Fisher Scientific) under 70 to 80% confluent conditions, the cells were collected as single cells. The cells were suspended in E8 medium containing 10 µM Y-27632 (ROCK inhibitor, Wako Pure Chemical Industries, Ltd.), and seeded at 1.2 to 1.5 x 10⁵ cells/cm² in a culture container coated with Geltex (Thermo Fisher Scientific). The 454E2 line was adhesion-cultured to induce differentiation into pancreatic progenitor cells. Differentiation induction was performed using the same culture solution for the same culture period as described above, except that culture of the fourth stage was performed for 3 days.

The 771-2 line was cultured in StemFit AK02N medium (Ajinomoto Co., Inc.) for 3 to 4 days using a culture container coated with Geltex (Thermo Fisher Scientific). After treatment using TrypLE (Thermo Fisher Scientific), the cells were collected as single cells. Then, the cells were suspended in StemFit AK02 medium (Ajinomoto Co., Inc.) containing 10 µM Y-27632 (ROCK inhibitor, Wako Pure Chemical Industries, Ltd.), and seeded at 1.2 to 1.5 x 10⁵ cells/cm² in a culture container coated with Geltex (Thermo Fisher Scientific). The 771-2 line was adhesion-cultured to induce differentiation into pancreatic progenitor cells. Differentiation induction was performed using the same culture solution for the same culture period as described above, except that culture of the fourth stage was performed for 3 days.

The P11025 line was cultured using DEF-CS Culture System (Takara Bio, Inc.) for 3 to 4 days. After treatment using TrypLE (Thermo Fisher Scientific), the cells were collected as single cells. Then, the cells were suspended in DEF-CS medium (Takara Bio, Inc.) containing 10 µM Y-27632 (ROCK inhibitor, Wako Pure Chemical Industries, Ltd.), and seeded at 1.2 to 1.5 x 10⁵ cells/cm² in a culture container coated with DEF-CS Coat (Takara Bio, Inc.). The P11025 line was adhesion-cultured to induce differentiation into pancreatic progenitor cells. Differentiation induction was performed using the same culture solution for the same culture period as described above, except that culture of the fourth stage was performed for 3 days.

### Amplification of Pancreatic Progenitor Cells Derived from Other iPS Cell Lines

The pancreatic progenitor cells obtained above were dispersed into single cells using a cell dispersion enzyme solution TrypLE (Thermo Fisher Scientific), as with the 253G1 line. The cells were suspended in the following medium containing 10 µM Y-27632 (ROCK inhibitor, Wako Pure Chemical Industries, Ltd.), and seeded at 1000 cells/well (1000 x 256 = 2.56 x 10⁵/plate) in a 256-well agarose microwell plate placed on a well of a 12-well plate. After the agarose microwell plate was left to stand for 10 minutes to precipitate the cells in the bottom, the following medium was added in the vicinity of the agarose microwell plate to immerse the plate in the medium. Thereafter, culture was performed for 4 days at 37°C with 5% CO₂. Medium replacement was performed every other day. Culture was also performed using media containing three factors (EGF + RSPD1 + CHIR99021 or EGF + RSPD1 + FGF-7) or two factors (FGF-7 + CHIR99021), in place of the medium containing four factors (EGF + RSPD1 + FGF-7 + CHIR99021).
MCDB131 + 1.5 g/L NaHCO₃ + 0.5% fat-free BSA + 1/200 ITS supplement + 2 mM GlutaMax + 20 mM D-glucose + 50 ng/mL epidermal growth factor (EGF, Wako Pure Chemical Industries, Ltd.) + 200 ng/mL r-spondin 1 (RSPD1, R&D Systems) + 0.25 µM SANT-1 (Wako Pure Chemical Industries, Ltd.) + 0.2 µM LDN193189 (Wako Pure Chemical Industries, Ltd.) + 0.1 µM retinoic acid (Sigma-Aldrich) + 4.5 µM CHIR99021 (Tocris Bioscience) + 50 ng/mL fibroblast growth factor 7 (FGF-7, PeproTech)

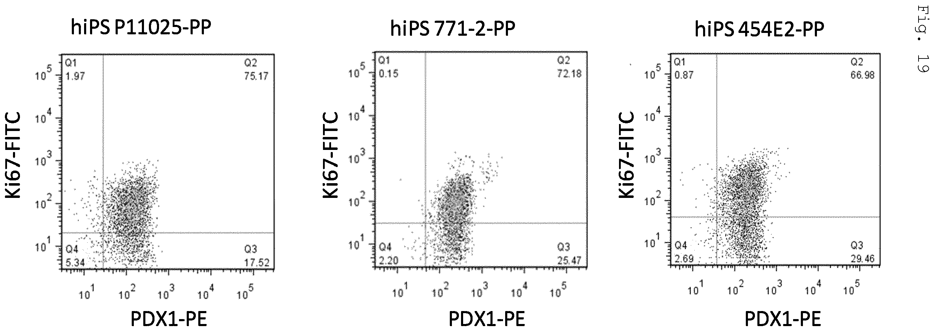

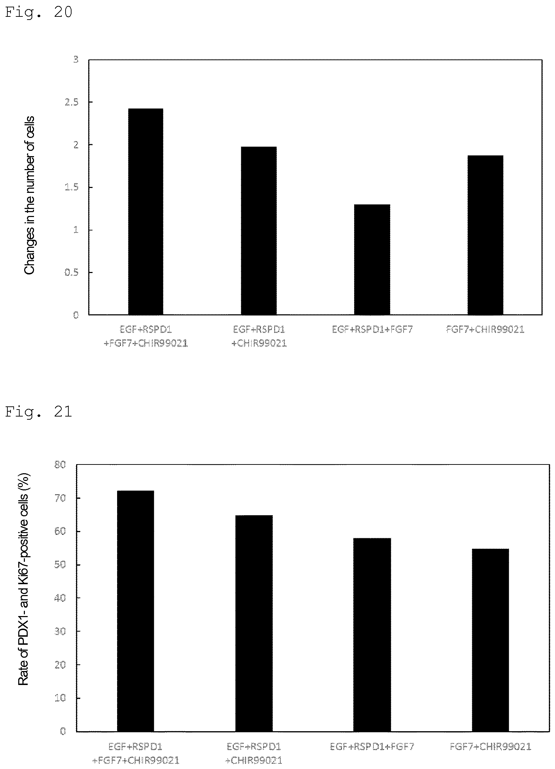

Figs. 18 to 21 show the results. Fig. 18 shows changes in the number of pancreatic progenitor cells derived from the 771-2 line when the four factors (EGF + RSPD1 + FGF-7 + CHIR99021) were added (cultured for 8 days). Due to the addition of the four factors, the pancreatic progenitor cells derived from the 771-2 line were proliferated about twice by culture for 4 days. Moreover, Fig. 19 indicates that in the three cell lines, about 70% of the cells were positive to a pancreatic cell marker PDX1 and a cell division marker Ki67 when the four factors were added. Figs. 20 and 21 show the results of adding two to four factors. These results demonstrate that the cells were proliferated about twice when the three factors (EGF + RSPD1 + CHIR99021 or EGF + RSPD1 + FGF-7) were added, and when the two factors (FGF-7 + CHIR99021) were added; that 50% or more of the cells were positive to PDX1 and Ki67; and that it was possible to proliferate the pancreatic progenitor cells.

After the pancreatic progenitor cells derived from the P11025 line were passaged twice in a growth medium to which the four factors (EGF + RSPD1 + FGF-7 + CHIR99021) were added, the cells were matured into endocrine cells. The mature culture method was the same as that for the pancreatic progenitor cells derived from the 253G1 line. As a result of immunostaining the differentiated cells, the cell aggregates differentiated into islet cells contained insulin-positive β-cells, glucagon-positive α-cells, and somatostatin-positive δ-cells.

All the publications, patents, and patent applications cited in the present specification are directly incorporated by reference into the present specification.

## Claims

1. A method for culturing pancreatic progenitor cells derived from pluripotent stem cells, the method comprising step (A) of three-dimensionally culturing pancreatic progenitor cells derived from pluripotent stem cells in a medium containing (1) a factor belonging to the epidermal growth factor (EGF) family and/or a factor belonging to the fibroblast growth factor (FGF) family, and (2) a Wnt agonist.

2. The method according to claim 1, wherein the Wnt agonist is a protein belonging to the R-spondin family and/or a GSK inhibitor.

3. The method according to claim 1, wherein the factor belonging to the EGF family and/or the factor belonging to the FGF family (1) is EGF, and the Wnt agonist (2) is R-spondin 1.

4. The method according to any one of claims 1 to 3, wherein the medium is a serum-free medium.

5. The method according to any one of claims 1 to 4, wherein the culture is culture in the absence of feeder cells.

6. The method according to any one of claims 1 to 5, wherein the pluripotent stem cells are iPS cells or ES cells.

7. The method according to any one of claims 1 to 6, wherein the pluripotent stem cells are derived from a human.

8. The method according to any one of claims 1 to 7, wherein the three-dimensional culture is suspension culture of aggregates of pancreatic progenitor cells.

9. The method according to any one of claims 1 to 8, further comprising step (B) of subculturing the pancreatic progenitor cells obtained in step A.

10. The method according to any one of claims 1 to 9 for use in purification of pancreatic progenitor cells.

11. The method according to any one of claims 1 to 10, further comprising step (C) of preparing iPS cells, wherein pancreatic progenitor cells derived from the iPS cells are used in step A.

12. The method according to any one of claims 1 to 11, further comprising step (D) of inducing the differentiation of pluripotent stem cells into pancreatic progenitor cells, wherein the pancreatic progenitor cells are used in step A.

13. A method for producing islet cells from pancreatic progenitor cells derived from pluripotent stem cells, the method comprising step (E) of inducing the differentiation of pancreatic progenitor cells cultured by the method according to any one of claims 1 to 12 into islet cells.

14. A method for cryopreserving pancreatic progenitor cells derived from pluripotent stem cells, the method comprising step (F) of freezing pancreatic progenitor cells cultured by the method according to any one of claims 1 to 12.

15. A medium for culturing pancreatic progenitor cells derived from pluripotent stem cells, the medium containing (1) a factor belonging to the EGF family and/or a factor belonging to the FGF family, and (2) a Wnt agonist.
